# FASCICULE DE BREVET EUROPEEN

(11) **EP 0 564 580 B1**
(45) Date de publication et mention de la délivrance du brevet: **06.09.1995**
(21) Numéro de dépôt: 92903531.9
(22) Date de dépôt: 27.12.1991
(51) Int. Cl.: A61F 2/02

(54) **DISPOSITIF FILTRANT DESTINE A LA PREVENTION DES EMBOLIES ET/OU LA DISTENSION DES PAROIS DES VAISSEAUX SANGUINS**
FILTER ZUM ZURÜCKHALTEN VON TROMBOEMBOLEN UND/ODER ZUM AUFSPREIZEN DER WANDUNGEN DES BLUTGEFÄSSES
FILTERING DEVICE FOR PREVENTING EMBOLISM AND/OR THE DISTENSION OF BLOOD VESSEL WALLS

(30) Priorité: 28.12.1990 FR 9016516
(43) Date de publication de la demande: 13.10.1993
(73) Titulaire: Laerum, Frode, N-0380 Oslo 3 (NO)
(72) Inventeur: Laerum, Frode, N-0380 Oslo 3 (NO)
(74) Mandataire: Lindgren, Anders
(86) Numéro de dépôt international: FR9101076
(87) Numéro de publication internationale: WO9211823

(56) Documents cités:
- EP-A- 0 201 466
- EP-A- 0 238 952
- EP-A- 0 323 333
- DE-A- 3 203 410
- FR-A- 2 645 731

## Description

La présente invention a pour objet un dispositif filtrant destiné à la prévention des embolies et/ou la distension des parois des vaisseaux sanguins.

On sait que les embolies résultent de l'oblitération d'un vaisseau sanguin par un corps étranger, comme par exemple un caillot, véhiculé par le sang, jusqu'au lieu où le calibre est insuffisant pour permettre son passage. Ainsi, les embolies pulmonaires sont provoquées généralement par les emboles provenant de thromboses ou des phlebites du membre inférieur, d'où ils se dirigent vers l'artère pulmonaire par la veine cave inférieure et la circulation cardiaque droite.

L'embolie pulmonaire est une complication courante chez les malades grabataires, notamment durant la période post-opératoire, ou chez les personnes âgées au décours de maladies inflammatoires, ou d'accidents vasculaires cérébraux. Elle peut également constituer un problème chez le malade jeune après traumatisme ou accouchement.

L'embolie pulmonaire est la cause directe de mortalité la plus répandue durant la période terminale de l'existence. L'embole est le plus souvent provoqué par une thrombose veineuse profonde (TVP) du membre inférieur. Les personnes ayant survécu à une embolie pulmonaire sont susceptibles de souffrir d'altérations profondes du système vasculaire pulmonaire et d'hypertension pulmonaire chronique aboutissant à la mort.

On observe des thromboses veineuses profondes chez un pourcentage de malades atteignant 60 % après chirurgie de la hanche, mais elles sont également fréquentes après d'autres interventions chirurgicales. C'est ainsi que les thromboses veineuses profondes constituent un problème clinique important dans les accidents vasculaires cérébraux, après infarctus du myocarde, dans certaines formes de cancer ou chez les malades présentant des troubles de coagulation sanguine.

Le traitement des thromboses veineuses profondes est dominé à l'heure actuelle par les médications systémiques utilisant l'héparine ou l'héparine-fragmine, en plus du traitement anti-coagulant par la coumarine.

Chez les malades jeunes et dans des cas graves, sélectionnés (par exemple thrombose de la veine pelvienne), il est possible d'utiliser également un traitement fibrinolytique par voie générale. Cependant, le traitement par voie générale n'est pas exempt de complications, et notamment d'hémorragies.

On a continué ces dernières années le traitement local, tel qu'exérèse chirurgicale des thrombus de la veine pelvienne, associée à une fistule artériovéineuse artificielle pratiquée dans l'aine et à un traitement anticoagulant. Les résultats sont variables, mais ce traitement n'a pas été adopté sur une vaste échelle.

Une thrombose veineuse profonde peut évoluer naturellement vers la lyse, ou aboutir à l'obstruction veineuse ou à la destruction de valvules veineuses. De tels états post-thrombotiques induisent à leur tour une insuffisance veineuse, une élévation de la pression veineuse périphérique, la formation d'anastosomes et de varices. L'effet final risque d'être très nuisible pour les malades et constitue un problème socio-économique important.

Par ailleurs, on a utilisé des filtres mécaniques dans la veine cave inférieure de malades présentant des thromboses veineuses profondes pour empêcher l'embole de migrer jusqu'aux artères pulmonaires.

De tels filtres sont généralement mis en place dans la veine de façon temporaire ou permanente, par voie percutanée haute (jugulaire) ou basse (fémorale) au moyen de cathéters ou de façon chirurgicale (plicature).

A l'heure actuelle, le filtre mécanique le plus utilisé est le filtre de Greenfield, constitué d'une pluralité de fils de forme ondulée se répartissant suivant un cône à partir d'un sommet commun.

D'autres filtres mécaniques tels que le filtre de Mobin-Uddin, le filtre de Gianturco, le filtre d'Amplatz, le filtre à panier de Günther sont également utilisés.

Bien que certains de ces filtres connus sont conformés de façon à pouvoir être retirés, leur enlèvement est généralement très difficile dans la pratique. Ils sont en outre susceptibles de se déplacer, de pénétrer dans la paroi veineuse ou de provoquer des réactions thrombotiques locales suivies d'obstruction de la veine cave.

Par ailleurs, dans les artères, la dissection des tuniques de la paroi est susceptible de donner naissance à de faux canaux qui obstruent les artères qui se ramifient. Il s'agit d'une maladie rare, mais à risque mortel, survenant brutalement.

On connaît également, notamment par les documents FR-2 625 437 et FR-2 632 864 de la Société BIOMAT et FR-2 541 901 de Monsieur Franceschi un dispositif filtrant comportant une partie active destinée à être placée sur le trajet sanguin constituée d'un fil en un matériau à mémoire de forme susceptible d'être amené dans une position sensiblement rectiligne et de reprendre, lorsqu'il est libéré, une forme correspondant à sa prédéformation dans laquelle il provoque une déformation du vaisseau sans arrêter le flux, en permettant ainsi de stopper la migration des gros caillots sanguins venus du réseau veineux périphérique.

Ces dispositifs présentent en position d'utilisation soit la forme de deux ellipses dont les grands axes forment entre eux un angle de 90° (FR-2 625 437 et FR-2 632 864), soit la forme d'ondulations principale et secondaire s'étendant dans des plans différents (FR-2 541 901 ou FR-2 645 731).

De tels dispositifs sont cependant difficiles à enlever et ne sont pas adaptés à une mise en place temporaire dans les vaisseaux.

La présente invention a donc pour but de résoudre le nouveau problème technique consistant en la fourniture d'un dispositif filtrant destiné à la prévention des embolies et/ou la distension des parois des vaisseaux sanguins d'une nouvelle conception, qui puisse être mise en place de façon permanente ou temporaire, facilement et sans nécessiter un guide de diamètre important.

La solution, conforme à la présente, pour résoudre ce problème technique, consiste en un dispositif filtrant, destiné à la prévention des embolies et/ou la distension des parois des vaisseaux sanguins, comme défini par les caracteristiques de la revendication 1

Cette solution permet de résoudre le problème technique précédemment énoncé, d'une manière extrêment simple, facile à mettre en oeuvre à l'échelle industrielle.

Cette conformation est particulièrement avantageuse, dans la mesure où l'on obtient ainsi un filtre de taille unique, qui peut être utilisé pour des vaisseaux de taille variable. En effet, comme on le comprend, la partie active étant réalisée sous la forme d'un ressort enroulé en spirale, on pourra introduire dans le vaisseau une portion seulement de cette spirale, pour une veine de petite taille, ou une partie plus grande de cette spirale pour une veine de plus grande taille.

Ainsi, ce filtre convient à un plus grand nombre de calibres veineux et son fonctionnement peut être optimisé de façon plus individualisée par une meilleure régulation de l'hémodynamique au site du filtre.

Selon un autre mode de réalisation de l'invention, le fil semi-rigide précité s'étend dans un plan sensiblement perpendiculaire à celui de la partie active précitée.

Ce mode de réalisation est notamment utile pour comprimer temporairement les tuniques, afin d'éviter la poursuite de la dissection ou pour comprimer l'entrée des faux canaux. De la sorte, on peut également utiliser le dispositif en spirale comme filtre plus traditionnel dans la veine cave.

L'invention sera mieux comprise et d'autres buts, caractéristiques et avantages de celle-ci apparaîtront plus clairement à la lecture de la description explicative qui va suivre, faite en référence aux dessins schématiques annexés donnés uniquement à titre d'exemples non limitatifs, et illustrant deux modes de réalisation actuellement préférés de l'invention, dans lesquels :
- la figure 1a est une vue schématique de face d'un dispositif filtrant selon un premier mode de réalisation de l'invention ;
- la figure 1b en est une vue latérale ;
- la figure 2a est une vue schématique de face d'un dispositif filtrant selon un second mode de réalisation de l'invention ;
- la figure 2b en est une vue latérale ;
- la figure 3a est une vue schématique en coupe longitudinale illustrant la mise en place d'un dispositif filtrant tel que celui représenté aux figures 1a et 1b, à l'intérieur d'un vaisseau de petite taille ; et
- la figure 3b est une vue semblable à la figure 3a illustrant la mise en place d'un même dispositif filtrant à l'intérieur d'un vaisseau de plus grande taille.

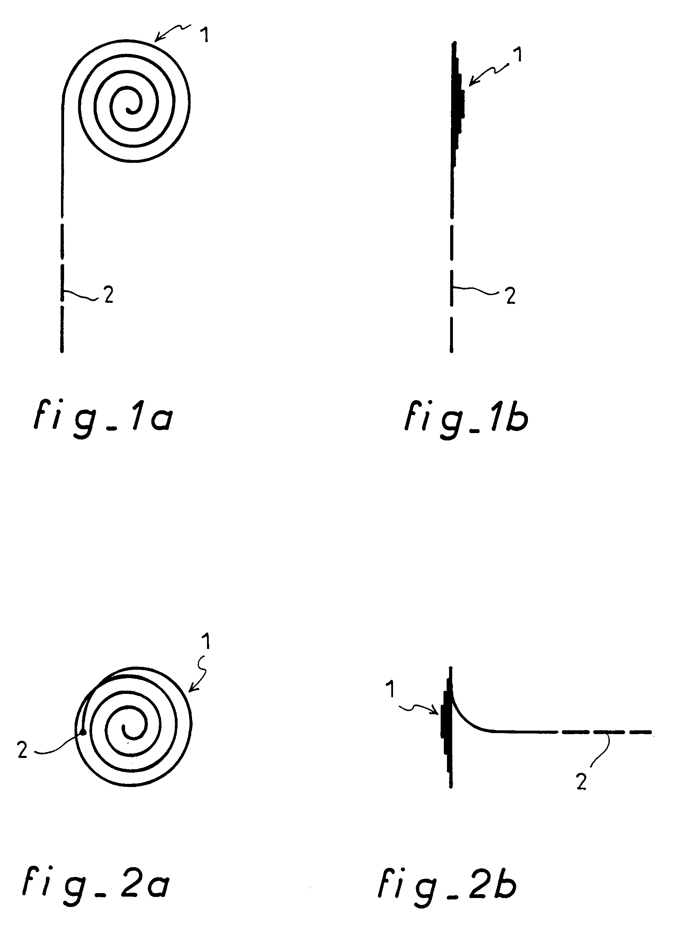

En se référant tout d'abord aux figures 1a, 1b, 2a et 2b, un dispositif filtrant (ci-après "filtre") conforme à la présente invention est essentiellement constitué d'une partie "active" 1, prolongée à l'extrémité par un fil flexible 2 sensiblement rectiligne destiné à introduire, retirer et fixer ladite partie active en position dans un vaisseau.

La partie active 2, destinée à être placée sur le trajet sanguin, est constituée d'un fil en un matériau à mémoire de forme susceptible d'être amené dans une position sensiblement rectiligne, pour être introduit dans un cathéter, et de reprendre, lorsqu'il est libéré dans le vaisseau, une forme correspondant à sa prédéformation.

Le matériau à mémoire de forme utilisé peut être métallique ou plastique. De préférence, le fil utilisé présente un diamètre compris entre 0,2 et 2 mm. Il est réalisé, par exemple en acier inoxydable de qualité médicale et présente un diamètre compris entre 0,2 et 1 mm. Il peut également être réalisé en matière plastique avec un diamètre compris entre 0,2 et 2 mm ou encore en acier inoxydable (0,05 à 1 mm) recouvert de matière plastique avec un diamètre de 0,2 à 1,5 mm.

Comme le montrent les figures 1a et 1b, le fil constituant la partie active présente, à l'état pré-déformé, la forme d'un ressort plat enroulé en spirale (ressort de montre). Ce fil est susceptible d'être amené dans une position sensiblement rectiligne, dans laquelle il peut être introduit dans le cathéter servant à la mise en place du filtre. Etant réalisé en un matériau à mémoire de forme, il reprend sa forme originale (ressort de montre) dès qu'il est hors du cathéter introducteur après positionnement dans le vaisseau sanguin.

Le fil flexible 2 peut être réalisé dans le même matériau que le fil constituant la partie active 1. Il peut également être constitué d'un matériau de nature différente.

Le fil 2 prolongeant la partie active 1 peut s'étendre dans le même plan que la partie active 1 (c'est-à-dire dans le plan du ressort de montre) comme le montrent les figures 1a et 1b.

Selon une variante de réalisation, le fil semi-flexible 2 peut être disposé sensiblement perpendiculairement au plan de la partie active comme le montrent les figures 2a et 2b.

La mise en place d'un dispositif filtrant, conforme à l'invention, à l'intérieur d'un vaisseau sanguin, peut être réalisée de façon connue en soi par poussée à l'aide du fil semi-flexible 2 dans une gaine d'introduction, préalablement positionnée dans le vaisseau dans lequel le dispositif doit être placé.

Les accessoires généralement nécessaires pour la mise en place comportent donc une gaine ou cathéter d'introduction dont le diamètre interne sera généralement d'environ 3 mm.

On prévoira également un moyen de fixation du dispositif filtrant, comme par exemple une épingle de sûreté semi-flexible fixée en un site distal.

Le dispositif filtrant représenté aux figures 1a et 1b convient à un nombre important de calibres veineux.

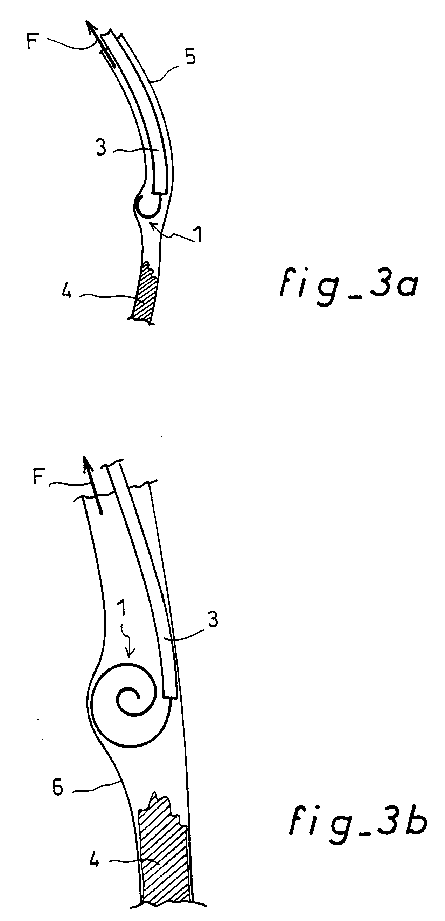

Ainsi, comme on le comprend en référence à la figure 3a, dans le cas d'un vaisseau 5 de petite dimension, comme par exemple une petite veine, on ne fera dépasser qu'une petite portion de la partie active de la pointe du cathéter introducteur 3, puis on accroche l'épingle de sûreté et le cathéter introducteur l'un à l'autre et on fixe le système au site d'introduction distal (à l'extérieur de la veine). Bien entendu, le dispositif filtrant est disposé au-dessus de la thrombose veineuse 4, de façon centrée (le flux sanguin étant représenté par la flèche F).

Pour un vaisseau sanguin 6 de plus grande dimension (figure 3b), on expulsera une plus grande portion de la partie active 1, en élargissant ainsi le diamètre total, avant de bloquer le système, afin d'obtenir les effets hémodynamiques souhaités au site du dispositif filtrant.

Dans sa version modifiée (figures 2a et 2b), le dispositif filtrant conforme à l'invention est utilisable pour distendre les parois artérielles disséquantes ou comme filtre veine-cave piégeant les emboles.

Le dispositif filtrant qui vient d'être décrit est notamment utile pour la prévention de l'embolie pulmonaire provoquée par des thrombus veineux et la facilitation de nouvelles méthodes thérapeutiques visant au traitement local de thromboses veineuses par des médicaments fibrinolytiques ou des thrombo-extracteurs mécaniques, par arrêt des caillots constituant un risque potentiel d'embolisation se déplaçant avec le flux sanguin, en donnant à la lumière veineuse une forme de fente au site du filtre, copiant une plicature veineuse.

Lorsque le risque d'embolie est jugé moins important, on déplie le filtre en le tirant vers l'arrière dans le cathéter introducteur et on le retire ainsi de l'organisme.

Un tel filtre présente de nombreux avantages.

Le princpal avantage est constitué par une possibilité permanente de retrait hors de l'organisme. Ainsi, lorsque le dispositif filtrant n'est plus nécessaire, on peut le retirer pour éviter les effets à long terme de corps étrangers (thrombose locale, inflammation, pénétration, hématomes périveineux).

Ce filtre, contraitement aux filtres veine-cave traditionnels, peut être utilisé pour des veines périphériques, c'est-à-dire les veines illiaque et fémorale. On évite par conséquent les effets ou les complications potentiels du filtre sur les deux membres lorsqu'un seul membre est atteint.

Dans sa première version, ce filtre convient à un grand nombre de calibres veineux, de sorte que son fonctionnement peut être optimisé de façon plus individualisée par une meilleure régulation de l'hémodynamique au site du filtre.

Dans sa seconde version, ce dispositif est utilisable pour distendre les parois artérielles diséquantes ou comme filtre veine-cave piégeant les emboles.

## Revendications

1. Dispositif filtrant, destiné à la prévention des embolies et/ou à la distension des parois des vaisseaux sanguins (5; 6), du types comportant une partie active (1) destinée à être placée sur le trajet sanguin, constituée d'un fil en un materiau à mémoire de forme, susceptible d'être amené dans une position sensiblement rectiligne et de reprendre, lorsqu'il est libéré, une forme correspondant à sa prédéformation, **caractérisé en** ce que, à l'état prédéformé, ladite partie active se présente la forme d'un ressort plat enroulé en spirale, et que celle-ci est prolongée au-delà de la spirale à son extrémité par un fil semi-flexible (2) destiné à introduire, retirer et fixer ladite partie active (1) en position de service dans le vaisseau sanguin (5; 6).

2. Dispositif selon la revendication 1, **caractérisé** en ce que le fil semi-flexible (2) précité s'étend dans le même plan que la partie active (1) précitée.

3. Dispositif selon la revendication 1, **caractérisé** en ce que le fil semi-flexible (2) précité s'étend dans un plan sensiblement perpendiculaire au plan de la partie active (1) précitée.

## Claims

1. Filtering device for preventing embolism and/or the distension of the blood vessel (5; 6) walls of the type comprising an active part (1) intended to be placed in the blood flow and in the form of a wire in a shape memory material, which can be brought into a substantially rectilinear position and which will regain a shape corresponding to its initial deformation when it is released, characterized in that said Active part, in its state of initial deformation, has the form of a flat spiral spring and is prolongated beyonf the spiral at its end by a semi-flexible wire (2), which is intended to introduce, retrieve and to secure said active part (1) in operating position in the blood vessel (5; 6).

2. Device according to claim 1, characterized in that said semi-flexible wire (2) extends in the same plane as said active part (1).

3. Device according to claim 1, characterized in that said semi-flexible wire (2) extends in a plane, which is substantially perpendicular to the plane of said active part (1).

## Patentansprüche

1. Filtrierende Vorrichtung zur Verhütung von Embolismus und/oder Ausdehnung von Wänden von Blutgefäßen (5; 6) vom Typ mit einem zum Verlegen im Blutfluß vorgesehenen, aktiven Teil (1), der die Gestalt eines in eine wesentlich geradlinige Position bringbaren und bei seiner Freigebung wieder eine der Anfangsdeformation entsprechende Gestalt annehmenden Drahtes aus Gedächtnismaterial aufweist, **dadurch gekennzeichnet**, daß der aktive Teil in seinem Zustand von Anfangsdeformation die Gestalt einer Spiralflachfeder aufweist und hinter der Spiral an seiner Ende durch einen halbnachgiebigen Draht (2), der zum Einführen, Wiedergewinnen und Sichern des aktiven Teils (1) in Betriebslage im Blutgefäß (5; 6) dient, verlängt ist.

2. Vorrichtung nach Anspruch 1, **dadurch gekennzeichnet**, daß der halbnachgiebige Draht (2) sich in derselben Ebene als der aktive Teil (1) erstreckt.

3. Vorrichtung nach Anspruch 1, **dadurch gekennzeichnet**, daß der halbnachgiebige Draht (2) sich in einer wesentlich quer zur Ebene des aktiven Teils (1) sich erstreckenden Ebene erstreckt.
